# EUROPEAN PATENT APPLICATION

(11) **EP 2 351 565 A1**
(43) Date of publication of application: **03.08.2011**
(21) Application number: 09821992.6
(22) Date of filing: 19.10.2009
(51) Int. Cl.: A61K 31/4245, A61K 31/421, A61P 29/00, A61P 31/22, C07D 409/12

(54) **MEDICINE FOR PREVENTING OR TREATING PAIN RELATED TO HERPES ZOSTER**

(30) Priority: 20.10.2008 JP 2008270329; 09.03.2009 JP 2009054932
(71) Applicant: Astellas Pharma Inc., Tokyo 103-8411 (JP)
(72) Inventor: SUZUKI, Hiroshi, Tokyo 103-8411 (JP); CHONO, Koji, Tokyo 103-8411 (JP); NOTO, Takahisa, Tokyo 103-8411 (JP); KATSUMATA Kiyomitsu, Tokyo 103-8411 (JP); NAKAMURA, Yoichi, Tokyo 103-8411 (JP)
(74) Representative: Bates, Philip Ian
(86) International application number: PCT/JP2009/067985
(87) International publication number: WO 2010/047295

(57) **Abstract**

[Problem] To provide a pharmaceutical composition for preventing or treating a zoster-associated pain.

[Means for Resolution] The inventors found that an N-{2-[(4-substituted phenyl)amino]-2-oxoethyl}tetrahydro-2H-thiopyran-4-carboxainide compound useful as a varicella-zoster virus (VZV) unexpectedly has the effect to prevent an irreversible nerve degeneration accompanied by herpes virus infection, and therefore is also useful as an agent for preventing or treating zoster-associated pain, and thus accomplished the present invention.

## Description

### TECHNICAL FIELD

The present invention relates to an agent for preventing or treating zoster-associated pain.

### BACKGROUND OF THE INVENTION

Herpes zoster is a viral disease in which varicella-zoster virus (VZV) is the causal virus. In general, initial infection with VZV occurs at the child stage, followed by onset as varicella, and at that time, virus establishes latent infection in the ganglion. When the latent VZV is recurred by its reactivation due to various factors, its onset as herpes zoster occurs (Arvin AM. Clini Microbiol Rev 1996; 9: 361 - 381). Though herpes zoster is rare in immunologically healthy young people, it is reported that its incidence rate increases with age and the incidence rate at from 50 to 80 years of age is 50% (Donahue JG. et al. Arch Intern Med 1995; 155: 1605 - 1609).
Clinical symptoms of herpes zoster are characterized that it is accompanied by zonal skin lesion which can be locally found in a characteristic certain innervation region and of a zoster-associated pain (ZAP). The skin lesion is naturally healed generally within about 3 weeks, but in the case of the aged, there is a tendency that it becomes severe and the period of time until its healing is prolonged. The ZAP is divided mainly into an acute stage pain and a chronic stage pain. Particularly, the chronic stage pain which continues over a prolonged period of time even after healing of the skin lesion is called postherpetic neuralgia (PHN) (Johnson RW. Herpes 2003; 10: 38 - 45).

While the acute phase pain of ZAP is based on the inflammation by a host-defense system reaction when infected with the virus, onset mechanism of the chronic stage pain is not thoroughly known. However, there is a report stating that atrophy of spinal dorsal horn, reduction of myelin and axon and lymphocyte infiltration in dorsal root ganglion and also reduction of axis cylinder in the skin epithelium were found in the body sides where herpes zoster was found in the case of PHN-suffered patients. The pain in the patients of chronic stage ZAP and PHN is considered to be neuropathic pain caused by the central and peripheral nervous system neural degeneration (Oaklander AL. Pain 2001; 92: 139 - 145, Watson CP. et al. Pain 1991; 44: 105-117, Rowbotham MC. et al. Neurobiol Dis 1996; 3: 205-214). It is known that incidence rate of PHN increases with age, and there has been reported that it is 21% at from 60 to 69 years of age, 29% at from 70 to 79 years of age and 34% at 80 years of age or higher (Hope-Simpson RE, et al. J R Coll Gen Pract 1975; 25: 571 - 575). With the aging in the future, it is expected that attacking case of herpes zoster will increase and the case of suffering from PHN will also increase.
It has been reported that acyclovir (ACV), valacyclovir (VACV) and famciclovir (FCV) as the existing anti-herpes agents which are used in the treatment of herpes zoster show the effect to accelerate healing of skin lesion and also shorten the duration of ZAP in some cases but cannot prevent onset of PHN completely (Wood MJ, et al. Clin Infect Dis 1996; 22: 341 - 347, Beutner KR, et al. Antimicrob Agents Chemother 1995; 39: 1546 - 1553, Tyring SK, Semin Dermatol 1996; 15: 27 - 31, Wood MJ, et al. N Engl J Med 1994; 330: 896 - 900, Rowbotham MC. Semin Nuerol 1994; 14: 247 - 254, Wood MJ, et al. Am J Med 1988; 85: 79 - 83, Huff JC, et al. Am J Med 1988; 85: 84 - 89, Mckendrick MW, et al. BMJ 1989; 298: 431). In addition, it has been reported by Oxman *et al*. that incidence rate of herpes zoster was reduced to about half by VZV vaccine, but evident effect was not found on the complication rate of PHN in the herpes zoster-suffering patients (Oxman MN, et al. N Engl J Med 2005; 352: 2271 - 2284). That is, even in the case of the agents which show therapeutic effect on the skin symptoms of herpes zoster, there are no agents which are expected to have an effect to prevent or treat ZAP and PHN, so that demand has been directed toward the development of a method for preventing or treating ZAP and PHN from the viewpoint of quality of life of patients.

Some amide compounds which have anti-viral effects on herpes simplex virus (HSV) and VZV have been reported (e.g., Patent References 1 to 8), and it has been disclosed that N-(4-methylphenyl)-N-(2-{[4-(1,3-oxazol-4-yl)phenyl]amino}-2-oxoethyl)tetrahydro-2H-thiopyran-4-carboxamide 1,1-dioxide and N-(2,6-dimethylphenyl)-N-(2-{[4-(1,2,4-oxadiazol-3-yl)phenyl]amino}-2-oxoethyl)tetrahydro-2H-thiopyran-4-carboxamide 1,1-dioxide particularly show good *in vivo* activity (e.g., Patent References 5 to 7). However, there are no reports about the effect of these compounds for preventing or treating ZAP and PHN.

### RELATED ART REFERENCES

### PATENT REFERENCES

Patent Reference 1: International Publication WO 97/24343
Patent Reference 2: International Publication WO 00/29399
Patent Reference 3: International Publication WO 02/38554
Patent Reference 4: International Publication WO 03/095435
Patent Reference 5: International Publication WO 2005/14559
Patent Reference 6: International Publication WO 2006/082820
Patent Reference 7: International Publication WO 2006/082822
Patent Reference 8: International Publication WO 2006/082821

### SUMMARY OF THE INVENTION

### PROBLEMS THAT THE INVENTION IS TO SOLVE

An object of the present invention is to provide an agent for preventing or treating ZAP or PHN.

### MEANS FOR SOLVING THE PROBLEMS

The present inventors found that an N-{2-[(4-substituted phenyl)amino]-2-oxoethyl}tetrahydro-2H-thiopyran-4-carboxamide compound is effective for the prevention or treatment of ZAP and PHN, and thereby accomplished the present invention.
That is, the present invention relates to a pharmaceutical composition for preventing or treating ZAP or PHN, which comprises an N-{2-[(4-substituted phenyl)amino]-2-oxoethyl}tetrahydro-2H-thiopyran-4-carboxamide compound represented by the formula (I) or a salt thereof as an active ingredient 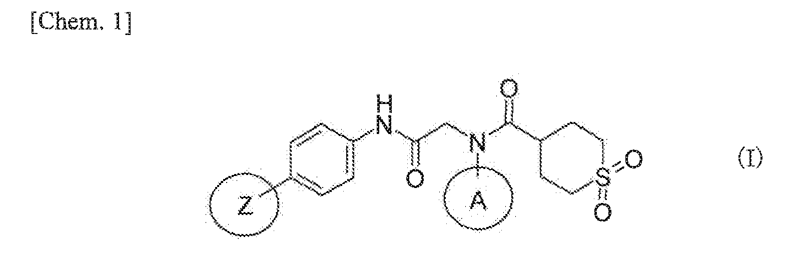 (in the formula, Z represents 1,2,4-oxadiazol-3-yl or 4-oxazolyl group, and A represents a phenyl group which is substituted with at least one methyl group and may further have one or two substituents selected from the group consisting of methyl group and halogen atoms, or 5-indanyl group).
Also, the present invention relates to a method for preventing or treating ZAP or PHN, which comprises administering an effective amount of the compound of formula (I) or a salt thereof. In addition, the present invention also relates to use of the compound of formula (I) or a salt thereof for the production of a pharmaceutical composition for preventing or treating ZAP or PHN and use of the compound of formula (I) or a salt thereof for preventing or treating ZAP or PHN.

### EFFECT OF THE INVENTION

The N-{2-[(4-substituted phenyl)amino]-2-oxoethyl}tetrahydro-2H-thiopyran-4-carboxamide compound or a salt thereof as the active ingredient of the pharmaceutical of the present invention is useful as an agent for preventing or treating ZAP or PHN.

### BRIEF DESCRIPTION OF THE DRAWING

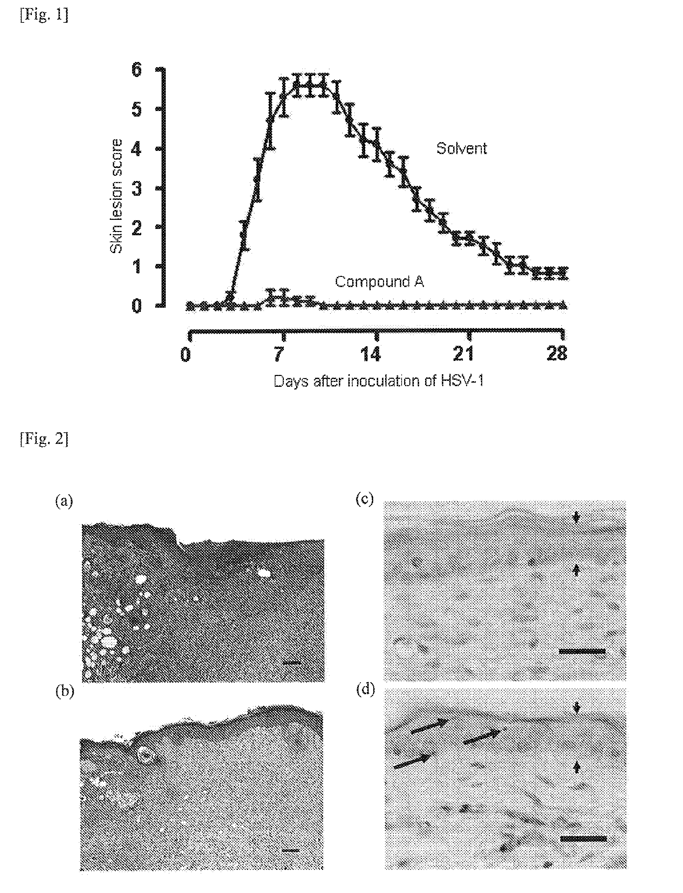

[Fig. 1] Fig. 1 shows a result of evaluation with time of skin lesion score after HSV-1 virus infection in Example 1 (N=10). The ordinate of the graph shows skin lesion scores, and the abscissa the number of days after HSV-1 inoculation. Closed circle represents skin lesion score of the solvent administration group, and closed triangle that of the compound A administration group.
[Fig. 2] Fig. 2 shows pathological images of skin section specimens at 11 and 28 days after the HSV-1 virus infection in Example 1. The image (a) is the skin at 11 days after infection in the solvent administration group, (b) is the skin at 11 days after infection in the compound A administration group, (c) is the skin at 28 days after infection in the solvent administration group and (d) is the skin at 28 days after infection in the compound A administration group. The (a) and (b) are HE staining and (c) and (d) are PGP 9.5 immunostaining. The area between short arrows indicates epidermis, and the long arrow shows a PGP 9.5 positive fiber in epidermis.

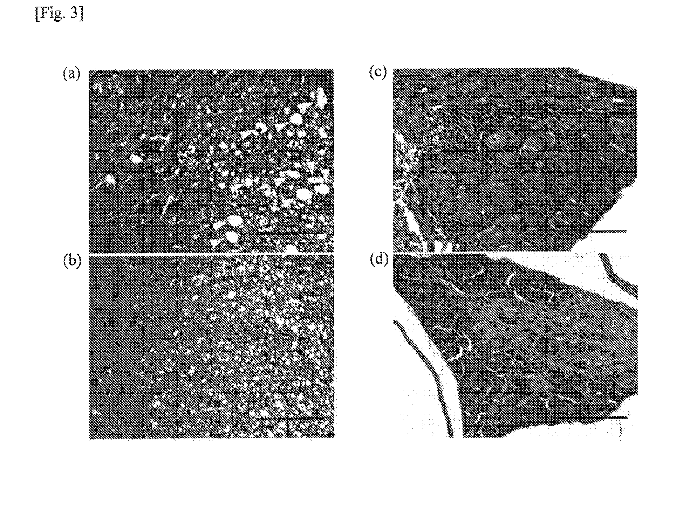

[Fig. 3] Fig. 3 shows pathological images of the spinal cord and dorsal root ganglia at 11 days after the HSV-1 virus infection in Example 1. The image (a) is the spinal cord at 11 days after infection in the solvent administration group (T4 level), (b) is the spinal cord at 11 days after infection in the compound A administration group (T6 level), (c) is the right dorsal root ganglion at 11 days after infection in the solvent administration group (T4 level) and (d) is the right dorsal root ganglion at 11 days after infection in the compound A administration group (T 6 level), and the arrow head shows the region where the nerve fiber vacuolated.

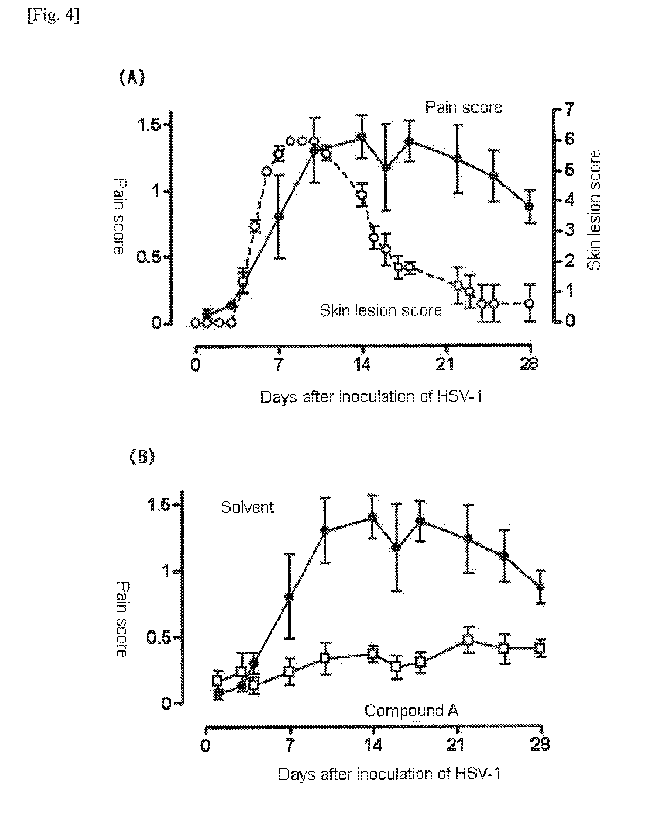

[Fig. 4] Fig. 4A is a graph showing a result of evaluation with time of skin lesion score and pain score after HSV-1 virus infection in Example 2 (N = 5). The right ordinate of the graph shows skin lesion scores and the left ordinate shows pain scores, and the abscissa shows the days after the HSV-1 inoculation. Closed circle represents pain scores and open circle skin lesion scores. Fig. 4B is a graph showing a result of evaluation with time of pain score after HSV-1 virus infection in Example 2 (N = 5). The ordinate of the graph shows pain scores, and the abscissa shows the number of days after HSV-1 inoculation. Closed circle represents scores of the solvent administration group, and open square that of the compound A administration group.

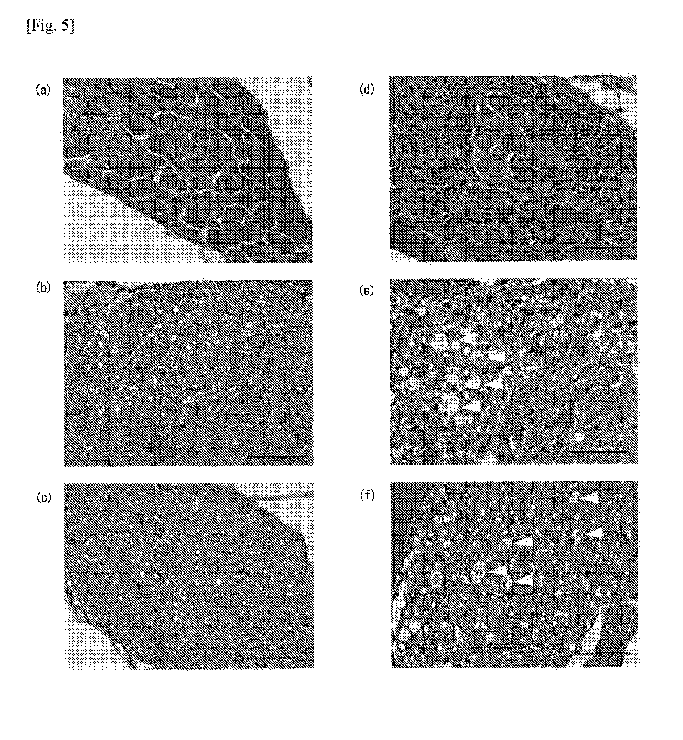

[Fig. 5] Fig. 5 shows pathological images of the right hind leg, spinal cord and dorsal root ganglia at 28 days after the HSV-1 virus infection in Example 2. The image (a) is the right dorsal root ganglion in the compound A administration group (L5 level), (b) is the spinal cord right dorsal horn in the compound A administration group (L5 level), (c) is the right hind leg sciatic nerve bundle in the compound A administration group, (d) is the right dorsal root ganglion in the solvent administration group (L4 level), (e) is the spinal cord right dorsal horn in the solvent administration group (L4 level) and (f) is the right hind leg sciatic nerve bundle in the solvent administration group, and the arrow head shows the region where the nerve fiber vacuolated.

### MODE FOR CARRYING OUT THE INVENTION

The N-{2-[(4-substituted phenyl)amino]-2-oxoethyl}tetrahydro-2H-thiopyran-4-carboxamide compound (to be referred sometimes to as "compound (I)" hereinafter) or a salt thereof as an active ingredient of the present invention is a compound disclosed in Patent Reference 5 and can be produced based on the descriptions in said Patent Reference and the aforementioned Patent References 3 to 5.
According to the present invention, F, Cl, Br and I atoms can be mentioned as the "halogen atom".
Also included in the compound (I) and a salt thereof are their hydrates and various solvates and polymorphic substances.

The "zoster-associated pain" and "ZAP" are pains which are generated accompanied by the onset of herpes zoster and include an acute stage pain and a chronic stage pain.
The "acute stage pain" means a pain during the period of from the initial stage of onset of herpes zoster to the skin lesion forming or healing stage, and the "chronic stage pain" means a pain in and after the skin lesion healing stage.
The "postherpetic neuralgia" and "PHN" means a chronic stage pain among the aforementioned chronic stage pains, which particularly continues over a prolonged period of time even after healing of the skin lesion.
The "prevention or treatment" includes a "treatment" which is used for the purpose of removing or alleviating said pain after onset of the acute stage pain and "prevention" which is used for the purpose of preventing onset or worsening of a pain before the onset or slight stage of the chronic stage pain.

Certain embodiments of the present invention are as follows.
(1) A pharmaceutical composition for preventing or treating ZAP, which comprises the compound (I) or a salt thereof as the active ingredient.
(2) A pharmaceutical composition for preventing PHN, which comprises the compound (I) or a salt thereof as the active ingredient.
(3) The pharmaceutical composition of the above-mentioned (1) or (2), wherein Z of the compound (I) is a 1,2,4-oxadiazol-3-yl group.
(4) The pharmaceutical composition of the above-mentioned (1) or (2), wherein Z of the compound (I) is a 4-oxazolyl group.
(5) The pharmaceutical composition of any one of the above-mentioned (1) to (4), wherein A of the compound (I) is a phenyl group which is substituted with at least one methyl group and may further have one or two substituents selected from the group consisting of a methyl group and halogen atoms.
(6) The pharmaceutical composition of any one of the above-mentioned (1) to (4), wherein A of the compound (I) is a 5-indanyl group.
(7) The pharmaceutical composition of the above-mentioned (1) or (2), wherein the compound (I) is a compound, or a salt thereof, selected from the group consisting ofN-(2,6-dimethylphenyl)-N-(2-{[4-(1,3-oxazol-4-yl)phenyl]amino}-2-oxoethyl)tetrahydro-2H-thiopyran-4-carboxamide 1,1-dioxide,
   N-(4-methylphenyl)-N-(2-{[4-(1,3-oxazol-4-yl)phenyl]amino}-2-oxoethyl)tetrahydro-2H-thiopyran-4-carboxamide 1,1-dioxide,
   N-(3-methylphenyl)-N-(2-{[4-(1,3-oxazol-4-yl)phenyl]amino}-2-oxoethyl)tetrahydro-2H-thiopyran-4-carboxamide 1,1-dioxide,
   N-(2-methylphenyl)-N-(2-{[4-(1,3-oxazol-4-yl)phenyl]amino}-2-oxoethyl)tetrahydro-2H-thiopyran-4-carboxamide 1,1-dioxide,
   N-(2,4-dimethylphenyl)-N-(2-{[4-(1,3-oxazol-4-yl)phenyl]amino}-2-oxoethyl)tetrahydro-2H-thiopyran-4-carboxamide 1,1-dioxide,
   N-(3,4-dimethylphenyl)-N-(2-{[4-(1,3-oxazol-4-yl)phenyl]amino}-2-oxoethyl)tetrahydro-2H-thiopyran-4-carboxamide 1,1-dioxide,
   N-(2,3-dihydro-1H-inden-5-yl)-N-(2-{[4-(1,3-oxazol-4-yl)phenyl]amino}-2-oxoethyl)tetrahydro-2H-thiopyran-4-carboxamide 1,1-dioxide,
   N-(4-chloro-3-methylphenyl)-N-(2-{[4-(1,3-oxazol-4-yl)phenyl]amino}-2-oxoethyl)tetrahydro-2H-thiopyran-4-carboxamide 1,1-dioxide,
   N-(3-fluoro-4-methylphenyl)-N-(2)-{[4-(1,3-oxazol-4-yl)phenyl]amino}-2-oxoethyl)tetrahydro-2H-thiopyran-4-carboxamide 1,1-dioxide,
   N-(3-fluoro-2,4-dimethylphenyl)-N-(2-{[4-(1,3-oxazol-4-yl)phenyl]amino}-2-oxoethyl)tetrahydro-2H-thiopyran-4-carboxamide 1,1-dioxide,
   N-(3,5-difluoro-4-methylphenyl)-N-(2-{[4-(1,3-oxazol-4-yl)phenyl]amino}-2-oxoethyl)tetrahydro-2H-thiopyran-4-carboxamide 1,1-dioxide, N-(2-fluoro-4-methylphenyl)-N-(2-{[4-(1,3-oxazol-4-yl)phenyl]amino}-2-oxoethyl)tetrahydro-2H-thiopyran-4-carboxamide 1,1-dioxide,
   N-(2,3-dimethylphenyl)-N-(2-{[4-(1,2,4-oxadiazol-3-yl)phenyl]amino}-2-oxoethyl)tetrahydro-2H-thiopyran-4-carboxamide 1,1-dioxide,
   N-(2,4-dimethylphenyl)-N-(2-{[4-(1,2,4-oxadiazol-3-yl)phenyl]amino}-2-oxoethyl)tetrahydro-2H-thiopyran-4-carboxamide 1,1-dioxide,
   N-(2,6-dimethylphenyl)-N-(2-{[4-(1,2,4-oxadiazol-3-yl)phenyl]amino}-2-oxoethyl)tetrahydro-2H-thiopyran-4-carboxamide 1,1-dioxide,
   N-(4-fluoro-2,6-dimethylphenyl)-N-(2-{[4-(1,2,4-oxadiazol-3-yl)phenyl]amino}-2-oxoethyl)tetrahydro-2H-thiopyran-4-carboxamide 1,1-dioxide,
   N-(2,3-dihydro-1H-inden-5-yl)-N-(2-{[4-(1,2,4-oxadiazol-3-yl)phenyl]amino}-2-oxoethyl)tetrahydro-2H-thiopyran-4-carboxamide 1,1-dioxide,
   N-(3-fluoro-4-methylphenyl)-N-(2-{[4-(1,2,4-oxadiazol-3-yl)phenyl]amino}-2-oxoethyl)tetrahydro-2H-thiopyran-4-carboxamide 1,1-dioxide,
   N-(4-chloro-3-methylphenyl)-N-(2-{[4-(1,2,4-oxadiazol-3-yl)phenyl]amino}-2-oxoethyl)tetrahydro-2H-thiopyran-4-carboxamide 1,1-dioxide, and N-(3-fluoro-2,4-dimethylphenyl)-N-(2-{[4-(1,2,4-oxadiazol-3-yl)phenyl]amino}-2-oxoethyl)tetrahydro-2H-thiopyran-4-carboxamide 1,1-dioxide.
(8) A method for preventing or treating ZAP, which comprises administering an effective amount of the compound (I) or a salt thereof to a patient.
(9) A method for preventing PHN, which comprises administering an effective amount of the compound (1) or a salt thereof to a patient.
(10) Use of the compound (1) or a salt thereof, for the manufacture of a pharmaceutical composition for preventing or treating ZAP.
(11) Use of the compound (I) or a salt thereof, for the manufacture of a pharmaceutical composition for preventing PHN.

The salts of compound (I) are the salts disclosed in the aforementioned Patent Reference 5 and included in the present invention with the proviso that these are pharmaceutically acceptable salts. As such salts, as acid addition salts, there may be mentioned illustratively, acid addition salts with inorganic acids such as hydrochloric acid, hydrobromic acid, hydroiodic acid, sulfuric acid, nitric acid, phosphoric acid, organic acids such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, aspartic acid, glutamic acid, and the like, also as salts with bases, salts with inorganic bases containing metals such as sodium, potassium, magnesium, calcium, aluminum, or salts with organic bases such as methylamine, ethylamine, ethanolamine, lysine, ornithine and ammonium salts and the like.

Depending on the kind of substituents, there is a case in which various isomers, for example, geometrical isomers such as cis-trans and the like and tautomers such as ketoenol and the like, are present in the compound (I), and separated forms and mixtures of these isomers are included in the present invention. Further, there is a case in which the compounds of the present invention have asymmetric carbon atoms so that isomers based on asymmetric carbon atoms can exist. Mixtures and isolated products of these isomers are included in the present invention. In addition, there is a case in which the compounds of the present invention form N-oxides depending on the kind of substituents, and these N-oxide forms are also included therein. Further, various hydrates and solvates and polymorphic substances of the compounds of the present invention are also included therein. In this connection, all of the compounds which are converted into the compounds of the present invention or salts thereof by undergoing metabolism in the living body, so-called prodrugs, are also included in the present invention. As the groups which form the prodrugs, there may be mentioned the groups described in Prog. Med., 5:2157 - 2161 (1985) and the groups described in "Iyakuhin No Kaihatsu (Development of Medicines)" Volume 7 "Bunshi Sekkei (Molecular Design)" pp.163-198, published in 1990 by Hirokawa Shoten.
The pharmaceutical composition of the present invention can be prepared as a preparation containing its effective ingredient, the compound (I) or a salt thereof, by a generally used method using medical carriers, excipients and the like which are generally used in said field.
The administration may be in the form of oral administration by tablets, pills, capsules, granules, powders, liquids and the like, or parenteral administration by injections such as for intraarticular, intravenous, intramuscular and the like use, suppositories, eye drops, eye ointment, liquids for transdermal use, ointments, patches for transdermal use, transmucosal liquids, transmucosal patches, inhalations and the like.

As the solid composition for oral administration by the present invention, tablets, powders, granules and the like are used. In such a solid composition, one or two or more active ingredients are mixed with at least one inert filler, for example lactose, mannitol, glucose, hydroxypropylcellulose, microcrystalline cellulose, starch, polyvinyl pyrrolidone, and/or aluminum magnesium silicate and the like. In accordance with the usual way, the composition may contain inert additives, for example, a lubricant such as magnesium stearate and the like, a disintegrating agent such as carboxymethylstarch sodium, a stabilizing agent and a solubilization assisting agent. The tablets or pills may be coated with a sugar coating or a film of a gastric or enteric substance.
The liquid composition for oral administration includes pharmaceutically acceptable emulsions, solutions, suspensions, syrups, elixirs and the like and contains a generally used inert diluent such as purified water or ethyl alcohol. In addition to the inert diluent, said liquid composition may contain an auxiliary agent such as a solubilizing agent, a moistening agent and a suspending agent and a sweetener, a flavor, an aromatic and an antiseptic.
The injections for parenteral administration include aseptic aqueous or non-aqueous solutions, suspensions and emulsions. As the aqueous solutions, for example, distilled water for injection and physiological saline are included. As the non-aqueous solutions, for example, there are propylene glycol, polyethylene glycol, a plant oil such as olive oil, alcohols such as ethanol, polysorbate 80 (official name) and the like. Such a composition may further contain a tonicity agent, an antiseptic, a moistening agent, an emulsifying agent, a dispersing agent, a stabilizing agent or a solubilization assisting agent. These are sterilized for example by filtration through a bacteria retaining filter, blending of a germicide or irradiation. In addition, these can also be used by producing a sterile solid compositions and dissolving or suspending them in sterile water or a sterile solvent for injection prior to their use.

Transmucosal preparations such as inhalations, transnasal preparations are used in the solid, liquid or semisolid form and can be produced in accordance with a conventionally known method. For example, a conventionally known filler, as well as a pH adjusting agent, an antiseptic, a surfactant, a lubricant, a stabilizer, a thickener and the like, may be optionally added. An appropriate device for inhalation or emission can be used in the administration. For example, using a known device such as a metered dose inhaler and the like or a sprayer, a compound can be administered as such or as a powder of prescribed mixture or as a solution or suspension by combining it with a pharmaceutically acceptable carrier. A dry powder inhaler and the like may be for use in the single or multiple administration, and a dry powder or a powder-containing capsule can be used. Alternatively, it may be in the form of a pressure aerosol sprayer which uses a proper gas as an appropriate propellant, for example chlorofluoroalkane, hydrofluoroalkane, carbon dioxide and the like.

Daily dose of the compound (I) is generally from about 0.001 mg/kg to 50 mg/kg, preferably from 0.01 mg/kg to 30 mg/kg, further preferably from 0.05 mg/kg to 10 mg/kg, per body weight in the case of oral administration, and the daily dose is approximately from 0.0001 mg/kg to 10 mg/kg, preferably from 0.001 mg/kg to 1 mg/kg, per body weight in the case of intravenous administration, respectively appropriately, and this is administered once a day or by dividing it two or more times. The dose is optionally decided in response to individual cases by taking symptoms, age, sex and the like into consideration. In addition, when used as an external preparation, desirable is an external preparation which contains from 0.0001% to 20%, preferably from 0.01% to 10%, of the compound of the present invention. This is administered once or several times a day to the local region in response to the symptoms.

Regarding administration of the compound (I) for treating and/or preventing zoster-associated pain, it is desirable to carry out the first administration immediately after onset of the herpes zoster and preferably to start it after within 120 hours, more preferably within 72 hours.

In addition, the compound (I) may be optionally used concomitantly with other drugs, and as the concomitantly usable drugs, there may be mentioned for example other anti-herpes virus agents ACV, VACV, FCV, penciclovir, vidarabine, BVDU (bromovinyldeoxyuridine), foscarnet, ganciclovir, analgesics for postherpetic neuralgia amitriptyline (tricyclic antidepressant), gabapentin (anticonvulsant), lidocaine, mexiletine (antiarrhythmic drug), capsaicin and anti-inflammatory-analgesics indometacin, ibuprofen, celecoxib.

### EXAMPLES

The following illustratively describes the present invention based on Examples. The present invention is not limited to the scope described in the following Examples.

### Example 1

Using mice, examination was carried out on the tissue damage and nerve degeneration of the skin, spinal cord and spinal cord dorsal root ganglion by HSV-1 infection. Under ether anesthesia, right side of the dorsal part of a hairless mouse (HOS: HR-1, female, 7 weeks of age at the time of infection) was scratched with a needle, and 15 µl/mouse of an HSV-1 virus liquid was added dropwise to the scratched part to be infected. While, 15 µl/mouse of a solvent instead of the HSV-1 virus liquid was added dropwise in the pseudo-infection group. The compound to be tested (N-(2,6-dimethylphenyl)-N-(2-{[4-(1,2,4-oxadiazol-3-yl)phenyl]amino}-2-oxoethyl)tetrahydro-2H-thiopyran-4-carboxamide 1,1-dioxide: hereinafter, "compound A") was used by dissolving in a 25% Cremophor (registered trademark) EL/25% polyethylene glycol 400 aqueous solution. By setting the administration liquid volume per one administration to 10 ml/kg, each initial administration was started one day after the HSV-1 infection. Thereafter, 50 mg/kg was repeatedly administered orally twice a day for 5 days.
The skin and spinal column (including spinal cord and dorsal root ganglion) were collected at 11 or 28 days after the infection and fixed with a 10% neutral buffer formalin liquid to prepare paraffin sections. Also, specimens of spinal cord and dorsal root ganglion were prepared on the transverse plane at the level of fourth thoracic vertebra (T4) and sixth thoracic vertebra (T6). Regarding the staining, immunohistological staining for PGP 9.5 as neurite-specific antigen was carried out on the skin sections, in addition to the hematoxylin-eosin staining (HE staining).
The skin lesions which bear resemblance to herpes zoster and are developed accompanied by HSV skin infection were classified based on the skin lesion critical degrees and systemic symptoms and scored as from 0 to 7, every day from 1 day to 28 days after the infection.
Score 0: no symptoms
Score 1: erythema, blister or scab at local region
Score 2: erythema, blister or scab at small region
Score 3: erythema, blister or scab at middle region
Score 4: strap-like blister or scab
Score 5: erosion accompanied by ulcer at middle region
Score 6: strap-like ulcer
Score 7: death or paralysis
A result of evaluation with time of the skin lesion score is shown in Fig. 1. Skin lesion score of the solvent administration group started to increase at 4 days after the infection and reached maximum at 9 days after the infection. Thereafter, skin lesion was improved with the lapse of days and almost healed by Day 28.
HE staining image of the skin of solvent administration group is shown in Fig. 2a. At 11 days after the infection, a strong inflammatory response accompanied by falling off of epidermis and infiltration of mononuclear cells was observed around the skin region of HSV-1 infection(Fig. 2a). A PGP 9.5, the marker showing nerve ending in the epidermis, was considerably reduced even at Day 28 when epidermis was almost completely regenerated (Fig. 2c). HE staining images of spinal cord and dorsal root ganglion are shown in Fig. 3. In the solvent administration group at 11 days after the infection, vacuolization of white matter was observed, indicating a damage of nerve axon or medullary sheath (Fig. 3a). Further, degeneration of nerve cell and infiltration of mononuclear cell were observed also on the right dorsal root ganglion (Fig. 3c). The skin lesions and pathological findings after virus infection observed in the above were similar to the findings observed in herpes zoster patients (Oaklander AL. Pain 2001; 92: 139 - 145, Watson CP, et al. Pain 1991; 44: 105 - 117, Rowbotham MC, et al. Neurobiol Dis 1996; 3: 205 - 214).
The compound A inhibited the onset of skin lesions which resemble the herpes zoster by HSV-1 infection (Fig. 1). In addition, the compound A inhibited fall off of epidermis and infiltration of mononuclear cell and accelerated regeneration of epidermis (Fig. 2b). As a result of the PGP 9.5 immunostaining of the skin at 28 days after the infection, PGP 9.5 positive image was observed on the epidermis clearly frequently in comparison with the solvent control group, thus revealing inhibition of the reduction of nerve ending (Fig. 2d). Further, the compound A protected nerve fiber in the spinal cord (Fig. 3b) and inhibited degeneration of nerve cell and infiltration of mononuclear cell in the right dorsal root ganglion (Fig. 3d).
Based on the above, it was confirmed that the compound A not only inhibits onset of a herpes zoster-like skin lesion by the HSV-1 infection but also has the effect to inhibit neurodegeneration. Since the compound A also has the anti-viral activity on VZV (Patent References 5 to 8), it is evident that the same effect can be obtained also on the VZV infection, so that it was confirmed that it is effective for the prevention of the irreversible neurodegeneration observed in herpes zoster patients.

### Example 2

Examination was made on the effect of compound A upon the pain accompanied by viral infection. The mouse HSV infection pain model system on which onset of the pain similar to ZAP including acute phase and chronic phase pains has been reported was used in the evaluation of pain (Takasaki I, et al. Pain 2000; 86: 95 - 101, Takasaki I, et al. Jpn J. Pharmacol 2000; 83: 319 - 326, Takasaki I, et al. Anesthesiology 2002; 96: 1168 - 1174, Sasaki A, et al. Neuroscience 2007; 150: 459 - 466). Briefly, an area of 5x5 mm of epidermis of the lower part of the right hind leg knee joint of a hairless mouse (HOS: HR-1, female, 7 weeks of age at the time of infection) was scratched with a bundle often 27G intracutaneous injection needles, and 1.5 x 10⁵ pfu/10 µL of an HSV-1 virus liquid was added dropwise thereto and spread thereon tobe infected. Starting from one day after the infection, the compound A (50 mg/kg) or a solvent (25% Cremophor (registered trademark) EL/25% polyethylene glycol 400 aqueous solution) was orally administered twice a day for 5 days (5 animals each). The skin lesions were classified based on the skin lesion critical degrees and systemic symptoms and scored as from 0 to 7 (cf. the above-mentioned Example 1). The von Frey hair (0.4 g) was used for the measurement of pain-associated reaction of the HSV-1 infection side hind leg. The mouse was put into a measuring cage (100 mm in width x 100 mm in height x 300 mm in length) and accustomed to the environment for 10 minutes or more, and then the von Frey hair was vertically applied to the right side hind leg sole for from 3 seconds to 5 seconds, in such a degree that it slightly bent, and this was repeated 6 times at intervals of several seconds. The pain reaction of this case was evaluated as follows.
Score 0: no reaction
Score 1: lifting of hind leg
Score 2: sharp evasion reaction and flinching of hind leg
The pain score of each individual was calculated by averaging six scores.
The spinal column (including spinal cord and dorsal root ganglion) and right side hind leg femur part (including sciatic nerve bundle) were collected on the final day of the observation, namely at 28 days after the infection. The collected tissues were fixed with a 10% neutral buffer formalin liquid to prepare paraffin sections. Specimens of spinal cord and dorsal root ganglion were prepared on the transverse plane at the level of fourth lumber (L4) and fifth lumber (L5). A specimen was further prepared on the transverse plane containing the femur part sciatic nerve bundle, and each of the specimens was treated with HE staining.
Daily changes in the skin lesion score and pain score of the solvent administration group are shown in Fig. 4A. A herpes zoster-like skin lesion was generated at 4 days after the infection, reached its maximum score at 8 days after the infection and then recovered with the lapse of days and almost healed after about 3 weeks. On the other hand, the pain score increased with the appearance of skin lesion, and a high degree pain continued even at 28 days after the infection which is after healing of the skin lesion. Since progress of the onset of this lesion and pain is equivalent to the case reported by Takasaki *et al*. (Takasaki I, et al. Anesthesiology 2002; 96: 1168 - 1174), it was considered useful as a test model for judging effect on ZAP in human.
When the compound A was administered, increase of pain score was considerably inhibited at both of the early stage of onset and complete recovery stage of the lesion (Fig. 4B). In addition, it was shown that nerve cell degeneration was inhibited in the right dorsal root ganglion, spinal cord right dorsal horn and right side hind leg femur part sciatic nerve bundle, at 28 days after the infection which is the period of onset of PHN-like chronic stage pain (Fig. 5). That is, it was confirmed that the compound A is useful as an agent for treating or preventing ZAP, because it inhibited induction of the acute stage pain and also inhibited onset of the PHN-like chronic stage pain which continues even after healing of skin lesion.

### EXAMPLE 3

The effect of compound A to prevent and treat zoster-associate pain in patients who underwent herpes zoster was clinically evaluated by monitoring the presence or absence of pain after administration of the compound A in the following manner. For the purpose, Numeric pain scale (11 points of from 0 (no pain) to 10 (a pain of more than this cannot be considered)) was used in the pain evaluation (Oxman MN, et al. N Engl J Med 2005; 352: 2271 - 2284).
Under informed consent with the persons themselves, 100 mg, 200 mg or 400 mg of the compound A was taken once a day for 7 days by each of the patients who underwent herpes zoster, and the pain was recorded by them day by day by the Numeric pain scale using a diary. From the diary record, the number of patients having pain scales of 3 or more on 91 days after the first day of drug administration were counted as the cases of PHN onset, and using the ratio based on the all persons to be rested as the PHN incidence rate, the effect of the compound A to prevent PHN was evaluated. As a result, the PHN incidence rate in the group which took 100 mg, 200 mg or 400 mg of the compound A was 2.0% (2 cases in 99 persons), 1.0% (1 case in 97 persons) or 3.2% (3 cases in 95 persons), respectively. Compound A inhibited the onset of PHN in 95% or more of the herpes zoster patients in the clinical evaluation.

As a result of the above-mentioned each test, it was confirmed that the compound A is useful for not only the skin lesion of herpes zoster but also the prevention or treatment of the ZAP and PHN caused by herpes zoster. Accordingly, it is evident that the compound (I) which has been reported to have the antiviral activity similar to that of the compound A is useful as the active ingredient of a pharmaceutical composition for preventing or treating ZAP and PHN.

### INDUSTRIAL APPLICABILITY

The N-{2-[(44-substituted phenyl)amino]-2-oxoethyl}tetrahydro-2H-thiopyran-4-carboxamide compound or a salt thereof as an active ingredient of the pharmaceutical of the present invention is useful as an agent for preventing or treating ZAP and PHN.

## Claims

1. A pharmaceutical composition for preventing or treating a zoster-associated pain, which comprises a compound represented by the formula (I) or a salt thereof as an active ingredient 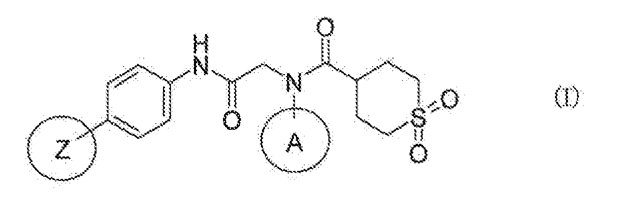 (in the formula, Z represents 1,2,4-oxadiazol-3-yl or 4-oxazolyl group, and A represents a phenyl group which is substituted with at least one methyl group and may further have one or two substituents selected from the group consisting of methyl group and halogen atoms, or 5-indanyl group).

2. The pharmaceutical composition described in claim 1, wherein the zoster-associated pain is postherpetic neuralgia.
